# EUROPEAN PATENT APPLICATION

(11) **EP 4 176 854 A1**
(43) Date of publication of application: **10.05.2023**
(21) Application number: 21315236.6
(22) Date of filing: 08.11.2021
(51) Int. Cl.: A61F 2/954, A61B 17/22, A61M 25/01

(54) **APPARATUS AND METHOD FOR DELIVERY OF AT LEAST ONE SURGICAL INSTRUMENT**

(71) Applicant: Caranx Medical SAS, 75013 Paris (FR)
(72) Inventor: SMITS, Jonas Victor Harmen, 3360 Bierbeek (BE); BERTHET- RAYNE, Pierre, 06800 Cagnes-sur-Mer (FR); sejor, Eric, 06000 Nice (FR)
(74) Representative: Hepp Wenger Ryffel AG

(57) **Abstract**

The invention relates to an apparatus (101) for delivery of at least one surgical instrument (10) in bifurcated body ducts of a patient. The apparatus (101) comprises an introducer unit (1) with a deployment member (2) for deploying surgical instruments (10). The deployment member (1) has at least one deflection section (7) for deploying, the surgical instrument (10) along an introductory path into a branching body duct. The apparatus comprises an adjustment element (3) configured to adjust an angular deployment direction of the surgical instrument (3) and to alter the pose of said at least one surgical instrument (10) with respect to the introducer unit (1).

## Description

The invention relates to an apparatus and a method for delivery of at least one surgical instrument into body ducts of a patient in a minimally invasive surgical procedure according to the independent claims.

In minimally invasive surgical procedures, in particular endovascular surgery, it is common to deploy multiple surgical instruments, especially guidewires.

Multiple surgical instruments are often required in a local region of interest for the delivery of multiple aortic stents or for the placement of cardiac sensing catheters in the heart for treating atrial fibrillation.

The surgical procedures are often time-consuming and require a great deal of experience and trial and error, as in many cases different surgical instruments have to be inserted sequentially into a patient's body ducts. The risk to the patient is strongly influenced by the duration of the surgical procedure, as well as the precision of the placement of the surgical instruments.

EP 1 363 558 B1 addresses some of these problems by providing a stent delivery system that can place guidewires in branched blood vessels through a lateral hole and a catheter body with a balloon. The system comprises a dual lumen catheter body for deploying a main vessel guidewire and a branch vessel guidewire.

However, the positioning of surgical instruments in branching body ducts remains difficult to perform precisely and without an extensive learning process, especially in a number of branching body ducts at steep angles.

It is an object of the present invention, to overcome the drawbacks of the prior art. In particular, the invention aims to provide a delivery apparatus which enables precise placement of the surgical instruments within a body duct with a low error rate, leading to a reduced time commitment due to reduced trial and error cycles. In addition, the apparatus should also be optimised so that no steep learning curve is required to operate the apparatus. In particular the surgical instruments shall be insertable into branching body ducts lateral to the apparatus reliably regardless of the orientation of the branching body ducts.

Moreover, the apparatus shall optimize the navigational complexity by optimising the procedural steps of the surgical procedure and also by increasing the flexibility during the placement of surgical instruments.

These and other objects are solved with an apparatus and a method according to the independent claims.

The apparatus according to the invention is suitable for delivery of at least one surgical instrument, in particular guidewires, into bifurcated body ducts of a patient. The apparatus comprises an introducer unit with a deployment member for deploying at least one surgical instrument. The deployment member has at least one deflection section for deploying the surgical instrument, in particular radially, along an introductory path into a branching body duct. The apparatus comprises an adjustment element configured to adjust an angular deployment direction of the surgical instrument at the deflection section and to alter a pose of said surgical instrument with respect to the introducer unit.

The adjustment element can act on the deflection section and thereby adjust the deployment direction.

Branching body ducts may in particular include arteries, preferably the aorta, and may include multiple branching body ducts which branch off from a main body duct, such as the renal arteries. The introducer unit is preferably substantially completely arrangeable in the main body duct.

The apparatus can be suitable for placement in bifurcated body ducts in a minimally invasive surgical procedure.

The angular deployment direction and the pose of said surgical instrument defines an introductory path for said surgical instrument, preferably along an angular deployment direction. The surgical instrument can be deployed by insertion along its longitudinal axis along an introductory path angle into the branched body duct relative to the introducer unit. The direction or angle of the introductory path is defined by the deflection section.

In one embodiment at least one surgical instrument, preferably multiple surgical instruments are pre-mounted within or on the introducer unit, preferably partially within the deployment member. A cavity within the introducer unit or on an outer surface of the introducer unit can be configured to receive at least one surgical instrument. The surgical instrument can be chosen from any type of currently available surgical instruments to fit the task at hand.

If there are several individual surgical instruments, they can preferably be operated independently of each other. Multiple surgical instruments can also be operated simultaneously in parallel such that the operating time is reduced.

The delivery of said surgical instruments can be carried out completely by a computer or aided by a computer.

The introducer unit is preferably elongated along its longitudinal axis. The introducer unit can be sized to extend over several branching body ducts along its longitudinal direction, in particular, over separate body ducts offset in the longitudinal direction of the introducer unit.

The introducer unit can either extend from the distal end of the apparatus to a proximal end region of the apparatus or extend from an intermediate region between the proximal end and distal end up to the distal end of the apparatus.

The introducer unit preferably has a longitudinal length, of 0.5 cm to 6 cm and a maximum radial extension of 0.2 cm to 0.8 cm.

The introducer unit can be deployable by a catheter and/or introduced through a catheter to the target location of bifurcated body ducts.

The apparatus can comprise a section proximal or distal to the introducer unit which has a smaller diameter than the introducer unit.

The dimensions of the introducer unit can be designed to fit the specific body passage for which the procedure is intended.

In some embodiments the adjustment element can have a guiding surface which is arrangeable in at least two different poses for guiding said at least one surgical instrument.

The guiding surface of the adjustment element can be configured to be pivoted around a first axis such as to change its angle relative to the longitudinal axis of the introducer unit at the deflection section. The guiding surface of the adjustment element can optionally be configured to be pivoted along a second axis in a different plane than the first axis of the introducer unit.

The guiding surface can be pivotable around an axis, which passes through the centre of the body of the guiding surface, through the outer edge of the guiding surface, or through an intermediate section of the guiding surface.

The guiding surface can be pivotable around the first axis such as to change its angle relative to the longitudinal axis of the introducer unit by 0° to 170°, in particular by 0° to 120°.

The guiding surface can be pivotable around the second axis such as to change its angle relative to the longitudinal axis of the introducer unit for a placement in a 360° azimuth range. Such a rotatable guiding surface preferably enables to alter the introductory path direction of the surgical instrument independently of the rotational position of the introducer unit.

In an alternative embodiment the alignment element may comprise multiple predetermined guiding surfaces or paths defining different poses, angular ranges and/or introductory paths. engaged by the surgical instrument by circumferentially adjusting the position of the alignment element.

The adjustment element can be translationally movable relative to the introducer unit in the longitudinal direction. A single adjustment element may then adjust the angular deployment orientation of more than one surgical instrument at different longitudinal locations.

The adjustment element can be positioned or positionable completely inside a cavity of the introducer unit.

The adjustment element can also be configured to partially extend in the radial direction from the introducer unit to provide an additional guiding surface into the branching body duct and/or reduce the lateral dimensions of the introducer unit. Such an increased radial dimension of the adjustment element might be advantageous to increase the size of the deployment section.

The apparatus can comprise a torsionally stiff section extending from the proximal end region of the apparatus to the distal end of the introducer unit.

With a torsionally stiff region a rotation by an angle results in a rotation of the distal end by substantially the same angle. The torsionally stiff region can allow for easier alignment of the introducer unit in relation to branching body ducts.

The alignment element may be configured automatically return to a predetermined position, if not actuated.

The predetermined position can be defined such that the friction transmitted to the surgical instrument is minimised when the apparatus is retracted.

The predetermined position can be adapted to the positions of the bifurcated body ducts or to an average angle of the body ducts.

The translational movement of the alignment element can allow adjustment of the pose and/or position of the surgical instrument along the longitudinal axis of the apparatus.

In some embodiments the deployment section can comprise a single opening or a slit elongated along an area of the outer surface of the introducer unit.

An alignment element completely positioned inside the introducer unit can provide a regular and smooth shape of the introducer unit independently of the orientation of the alignment element.

The alignment element preferably only extends over a fraction of the length or circumference of the introducer unit.

The adjustment element can be provided with an actuator, in particular actuated by (i) a microfluidic unit, (ii) an electromagnetic actuator, (iii) a micro-electro-mechanical system, (iv) a retractable pull-wire, (v) a volume adjustment unit, or (vi) a piezoelectric element.

The actuator is preferably configured to induce the rotational and/or translational movements of the alignment element.

Mechanical actuators can be based on springs which can be released.

A fluid operated volume adjustment unit can comprise conventional hydraulic or pneumatic cylinders, or an inflatable structure. The inflatable structure can alter the shape or the orientation of the guiding surface or deflection section size. The actuation may be due to an increase (inflation) or a decrease (vacuum) of volume of the inflatable structure.

The volume adjustment unit can be configured to generate a negative pressure, in particular a vacuum, within a non-compressible pressure chamber of the volume adjustment unit.

The piezoelectric element can be configured to apply a quasistatic spring force under load. The piezoelectric element may consist of stacked or individual multilayers to allow operating at low voltages.

The piezoelectric element can comprise layers with opposite poling vectors such that one layer can contract while the other layer can expand to form a piezoelectric bender actuator.

The actuator can be located within the introducer unit or at least partially at the proximal end region of the apparatus, in particular outside a patient's body.

Preferably, the actuator is designed in such a way that a continuous adjustment of the angular deployment orientation is possible.

The actuator can be operated manually or mechanically and automatically from a proximal end region of the apparatus.

The microfluidic unit may comprise microfluidic channels which can be operated by a saline solution so that even in the event of a defect, there is no danger to the patient.

In one embodiment the microfluidic channel can be nanofiber-reinforced and comprise or consist of a nanofiber network. The microfluidic channel can comprise or be made of a flexible polymer, in particular polydimethylsiloxane.

The volume adjustment unit can comprise a fluid reservoir in proximity of the adjustment element or can be supplied with fluid from a proximal end region of the apparatus.

The actuator can comprise means for exerting a force, in particular a rotational or translational force, on the adjustment element.

If an electromagnetic actuator is used, it can comprise a force sensor.

The deployment member can comprise an advancement unit for advancing the surgical instrument along an axis of the introducer unit. The advancement unit can be (i) an inchworm device for actively advancing the surgical instrument or (ii) a low friction elongated channel leading from a proximal end region of the apparatus to at least the deflection section of the apparatus. The low friction channel allows an advancement caused to the instrument at a proximal end, e.g. manually or mechanically. The apparatus can further have a manual or mechanical insertion unit which is configured for insertion of surgical instruments along said elongated channel.

The inchworm device can have a microfluidic mechanism or a piezoelectric mechanism driving the inchworm device.

The advancement unit can be configured to also retract the at least one surgical instrument.

The surgical instrument can be deployed from the deployment section into the branching body duct by moving it along the channel of the advancement unit. In this case, the deflection section may be part of the channel of the advancement unit.

The channel can be configured such that the at least one surgical instrument is arranged on an outer surface of the introducer unit and can be radially deployed out of the channel such that the introducer unit can be retracted after radial deployment.

The low friction elongated channel can have a fixation element having an activated and deactivated state. When activated the fixation element can hinder an axial movement of the at least one surgical instrument and when deactivated the fixation element can allow an axial movement of the surgical instrument along the channel. The fixation element can preferably be activated during introduction and/or retraction of the introducer unit such that the surgical instrument remains in a defined position.

The proximal end region of the apparatus can be placed outside the patient's body. The elongated channel preferably spans from the proximal end region up to the deployment section of the introducer unit.

The surgical instrument can be pre-positioned in the elongated channel.

The low friction can be provided with a coating of the channel with a low friction material. The surface of the channel can comprise or consist of siloxane or polytetrafluoroethylene.

The apparatus can comprise a retaining mechanism configured to releasably retain at least a section of said surgical instrument at the introducer unit prior to deployment. The retaining mechanism can be convertible from a retaining configuration to a release configuration.

The retaining mechanism may be formed by the low friction channel. In the retaining configuration the retaining mechanism can then be configured such that an elongated longitudinal section of the deployment member is at least partially enclosed in a circumferential direction of the introducer unit. In the release configuration the retaining mechanism can be configured such that the longitudinal section of the deployment member is at least partially open such that at least a portion of the surgical instrument confined within a cavity of the deployment member is detachable from the introducer unit in a radial direction of the introducer unit from the longitudinal section of the deployment member.

The retaining mechanism may be configured to retain the at least one surgical instrument at the introducer unit during the procedure of introducing the introducer unit and/or axially advancing the instrument for positioning or aligning the surgical instruments into branching body ducts.

The retaining mechanism generally can retain the surgical instrument by exerting a force on the at least one surgical instrument and/or by retaining the at least one surgical instrument within a recess or channel of the introducer unit.

The retaining mechanism can have a deformation area for retaining the surgical instrument, in particular a deformable section of the low friction channel. The deformation area can be deformable, in particular elastically deformable. The retaining mechanism can be convertible from the retaining configuration to the release configuration by applying pressure to the deformation area.

The retaining mechanism can be converted by applying a pressure supplied from a pressure reservoir which is positioned within the introducer unit or from an external source which can be located outside a patient's body.

Alternatively, the retaining mechanism can comprise at least one magnetic element which can retain the at least one surgical instrument and release the at least one surgical instrument by movement of the magnet relative to the surgical instrument.

The magnetic element can in particular comprise or consist of an electromagnet which can be switched on and off to be converted from the retaining configuration to the release configuration.

The retaining mechanism can alternatively comprise an adhesive which is connecting the surgical instrument to the outer surface of the introducer unit. The adhesive can be bioresorbable such that the surgical instrument can be detached from the introducer unit after or during the positioning in the branching body ducts. The adhesive can be configured such that the detachment of the surgical instrument is completed after a predetermined time interval.

The adhesive can be loosened by a relative movement of the retaining mechanism and/or introducer unit relative to the surgical instrument.

A single relative movement of the retaining mechanism and/or introducer unit can deactivate one common retaining mechanism such that multiple surgical instruments are detached in a single relative movement, preferably simultaneously.

The elongated longitudinal section of the deployment member can extend over a subsection of the introducer unit or preferably along the entire length of the introducer unit.

The longitudinal section can be arranged completely parallel to the introducer unit. However, an axis of the longitudinal section of the deployment member can also be configured to form an at least partially helical shape around the longitudinal axis of the introducer unit.

The outer diameter of the introducer unit preferably does not increase when the retaining mechanism is converted from the retaining configuration to the release configuration.

If a surgical instrument is not arranged on the other surfaces but within the introducer unit, sections of the surface of the introducer unit may be configured to provide an opening such that the introducer unit can be retracted after radial deployment.

The deflection section can have an opening in a lateral wall of the introducer unit allowing the deployment of said at least one surgical instrument from an inner cavity of the deployment member along the introductory path. A section of the introducer unit, preferably a section arranged at least arranged distally from the opening, can preferably provide a retraction recess in a longitudinal direction.

A retraction recess can e.g. be closed or opened by sections which are moveable and preferably rotatable relative to each other such as to partially or fully open the surface of the introducer unit along its length. In this case the retaining mechanism is formed by the closed outer surface of the introducer unit and can be brought from the retaining configuration to the release configuration by partially or fully opening the retraction recess in the surface of the introducer unit.

Several sections of the surface of the introducer unit may then move relative to each other, in particular in a circumferential direction, to be converted from the retaining configuration to the release configuration.

The retaining mechanism can in this case be convertible from the retaining configuration to the release configuration by (i) moving a section of the surface of the introducer unit, closing or opening an opening in a section of the surface of the introducer unit, and/or
(ii) pivoting a panel closing an opening in a section of the surface of the introducer unit about an axis, and/or
(iii) translationally moving at least one panel closing an opening in a section of the surface of the introducer unit.

The apparatus can comprise at least two deployment sections which are positioned at different circumferential positions of the introducer unit and/or at different longitudinal positions of the introducer unit.

The apparatus can comprise at least one radiopaque marker, preferably at the deployment section.

The radiopaque markers can be arrangeable corresponding to bifurcations of the body duct. The radiopaque markers can be arranged and/or formed such that angular orientation and/or longitudinal orientation of the deployment section in relation to the introducer unit can be retrieved by imaging techniques.

If there are more than one surgical instruments, the deployment member can have an individual mounting member which receives an surgical instrument, such that surgical instruments are pre-mountable relative to the respective deployment section.

The apparatus can comprise a stent mounted on a stent support. The stent can have fenestrations aligned with the deployment section.

The stent can be arranged coaxially with the introducer unit. The fenestrations of the stent preferably form passages in the radial direction of the stent.

The stent can comprise a wire mesh or a monolithic structure which is preferably at least partially tubular with openings at both ends.

The stent can be convertible between an expanded state and a collapsed state. The expanded state is preferably configured to frictionally engage the interior surface of the body duct, in particular the main body duct. The collapsed state is preferably configured to be attached or attachable to the introducer unit.

The surgical instrument and/or the at least one opening in the lateral wall for deploying the instrument for an inner cavity can be arranged in a pre-defined position in relation to the fenestrations of the stent.

The fenestrations of the stent may have dimensions corresponding to the cross-section of the branching body duct or body ducts. The fenestration in the expanded state of the stent can have substantially the same size as the opening of the body duct branching from the main body duct.

The longitudinal length of the stent can span at least one, in particular multiple, bifurcations of branching body ducts.

In one embodiment, the stent can be extended with further stent components which at least partially protrude into the branching body ducts and preferably are connectable to the stent. The further stent components are preferably configured to contact or frictionally engage the branching body duct wall.

The deployable surgical instruments, in particular guidewires, catheters and/or delivery devices, can be used during deployment of said further stent components, in particular after removal of the introducer unit.

The shortcomings of the state of the art are further addressed by a method for delivering at least one surgical instrument into bifurcated body ducts of a patient, optionally using an aforementioned apparatus. The method comprises the following steps:
Optionally, the location of the surgical instrument, preferably a plurality of surgical instruments and/or at least one opening in a lateral wall of an introducer unit, are pre-defined with respect to fenestrations of a stent placed or to be placed adjacent the bifurcated body duct.

A delivery apparatus is then inserted into a patient to a bifurcated body duct target location.

An angular orientation of said surgical instrument is then adjusted at a deployment section, preferably by actuating an adjustment element.

The surgical instrument is deployed, preferably radially, by using a deployment member along an introductory path along a body duct branching from the body duct in which the introducer unit is positioned.

Optionally, the surgical instrument may be detached by releasing a retaining mechanism.

The apparatus is then retracted from the body duct relative to the surgical instrument which remains at least partially within a branched off body duct.

The stent can be placed in advance of the surgical instrument using the introducer unit. The stent can be placed simultaneously, optionally by the same retaining means, with the surgical instruments. The stent can also be already deployed prior to the delivery of the at least one surgical instrument into the bifurcated body duct.

The invention will now be described with reference to specific embodiments and the accompanying drawings which show:
- Figure 1:: A schematic representation of an apparatus according to the invention,
- Figure 2:: the apparatus according to Fig. 1 with varying angular deployment orientations,
- Figure 3:: an apparatus with a pivotable adjustment element,
- Figure 4:: the embodiment according to Fig. 3 with a schematic advancement unit and retaining mechanism,
- Figure 5:: a cross-section in a plane perpendicular to the apparatus of an embodiment of the apparatus with a retaining mechanism in the retaining configuration,
- Figure 6:: the apparatus of Fig. 5 with a retaining mechanism in the release configuration,
- Figure 7:: an apparatus with an adjustment element which is rotatable about a first and a second axis,
- Figure 8:: an apparatus with a retaining mechanism partially enclosing the deployment member,
- Figure 9:: an apparatus with a retaining mechanism with two pivoting panels closing an opening,
- Figure 10:: a section of the outer surface of the introducer unit of inwardly pivotable panels,
- Figure 11:: a section of the outer surface of the introducer unit with a panel rotatable around its own geometric centre axis,
- Figure 12: the apparatus with an introducer unit according to Fig. 3 and Fig. 4 arranged inside a schematic bifurcated body duct,
- Figure 13:: the schematic bifurcated body duct of a patient,
- Figure 14:: an embodiment of the apparatus with a stent neighbouring the bifurcated body duct,
- Figure 15:: the embodiment of the apparatus according to Fig. 14 with a deployed stent,
- Figure 16:: the embodiment of the apparatus according to Fig. 14 and Fig. 15 with surgical instruments advanced to the location of the branching body ducts,
- Figure 17:: the embodiment of the apparatus according to Fig. 14 to Fig. 17 with a surgical instrument introduced at an angular orientation into the branching body duct,
- Figure 18:: the deployed surgical instruments and stent after retracting the apparatus according to the embodiment of Fig. 14 to Fig. 17,
- Figure 19:: a deployed stent in a bifurcated body duct connected with further stent components,
- Figure 20:: an embodiment of a deployed stent extending over an aneurism assuming a bent shape,
- Figure 21:: an illustration of the separate steps of a mechanism for advancing an instrument.

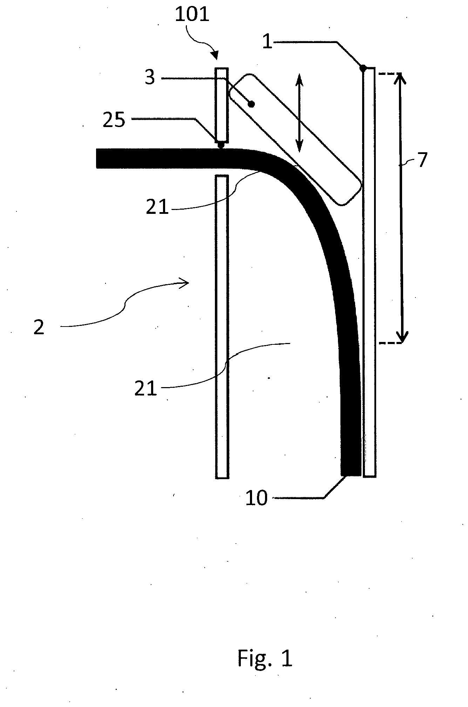

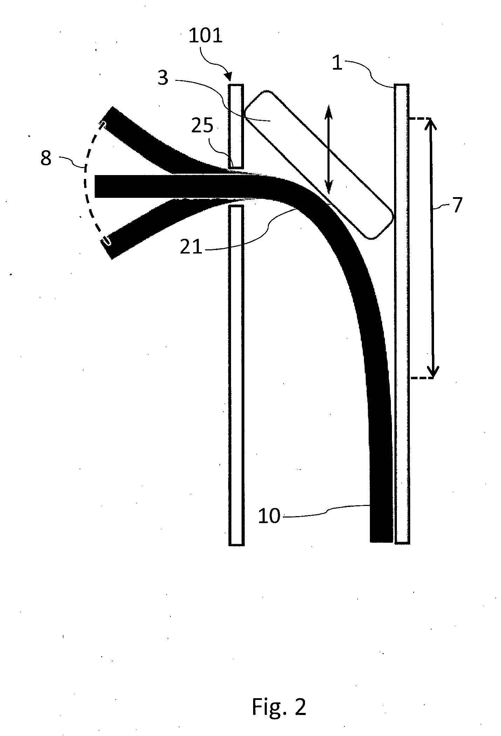

Figure 1 and Figure 2 show a subsection of an embodiment of an apparatus 101 along a longitudinal cross-section.

An outer surface of an introducer unit 1 encloses an adjustment element 3. A guidewire 10 is arranged within a deployment member 2 in the introducer unit 1.

The guidewire 10 can be advanced in its longitudinal direction following an introductory path and can be deployed through an opening 25 in the wall of the deployment member 2. The angle of the introductory path can be adjusted by the adjustment element 3.

A deflection section 7 denotes the section of the apparatus 101 in which the guidewire 10 can be bent. In order to allow the guidewire 10 to perform a bend which nevertheless does not lead to kinking and in particular minimises resistance to translation in the longitudinal direction of the guidewire 10, the guidewire 10 is arranged within the introducer unit 1 on the opposite lateral side than the opening 25 located on the outer surface of the introducer unit 1.

The radial opening 25 of the apparatus 101 has a size such that the guidewire 10 guided through it and be radially deployed. The guidewire can be advanced through the opening 25 in longitudinal direction.

In this embodiment the lateral opening 25 is a single hole positioned at a position to be arranged at a branching body duct.

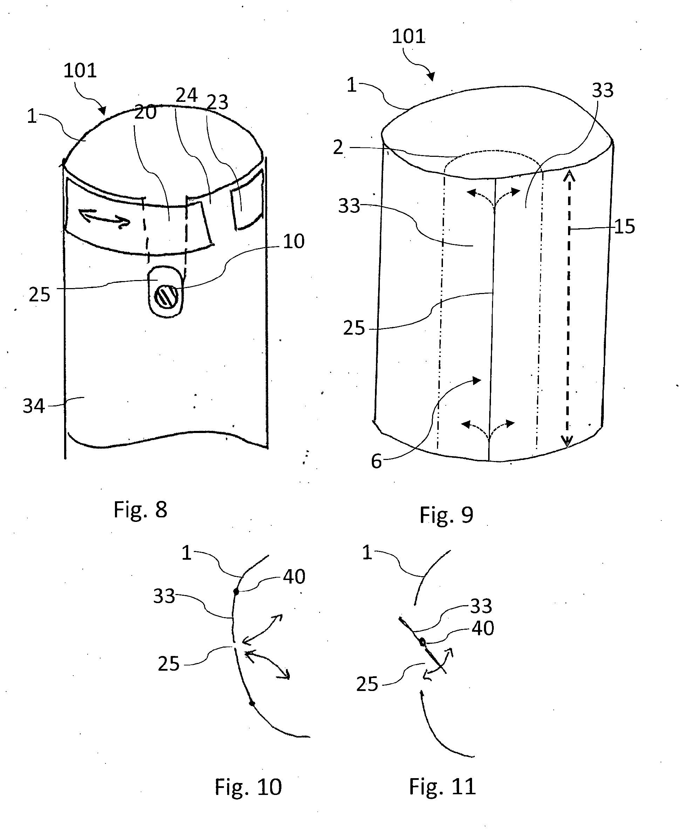

The opening 25 can comprise an open and closed configuration (not shown in Fig. 1 and Fig. 2). Furthermore, a retraction recess facilitating withdrawal of the introducer unit 1 after deployment can be provided, as will be shown in Fig. 8.

The adjustment element 3 in this embodiment is movable to adjust an angular deployment orientation 8 (see Fig. 2) of the guidewire.

The adjustment element 3 is translationally movable in the longitudinal direction of the introducer unit 1 in order to alter the deployment orientation 8 and/or the pose of the guidewire 10 as shown in Fig. 2.

The opening 25 serves as a contacting area for the guidewire which, together with the adjustment element 3, defines the angular deployment orientation and/or pose of the guidewire.

Figure 2 shows that a translational movement of the adjustment element 3 in a proximal direction of the apparatus 101 causes the guidewire 10 to bend towards a distal direction of the apparatus 101. A translational movement of the adjustment element 3 in the distal direction of the apparatus 101 causes the guidewire 10 to bend towards the proximal direction.

A guiding surface 21 of the adjustment element 3 contacts the guidewire 10 to deflect and guide it through the opening 25.

The adjustment element 3 can be operated manually or mechanically in an automated manner.

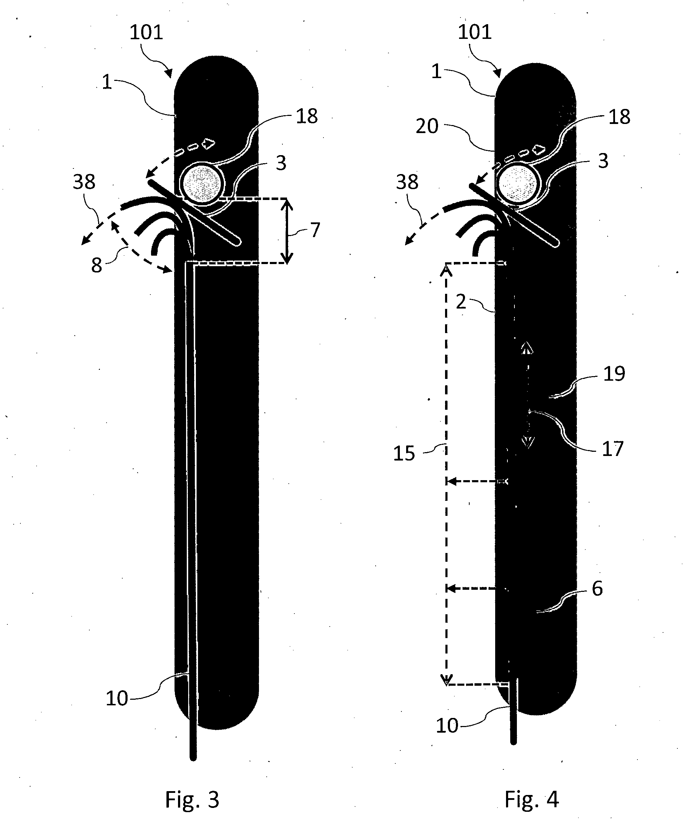

Figure 3 and figure 4 show a longitudinal cross-section of another embodiment of an apparatus 101 with a pivotable adjustment element 3 arranged partially within an introducer unit 1. The adjustment element 3 is pivotably mounted on an axis and a deployment orientation 8 can be adjusted within a deflection section 7 by pivoting the adjustment element 3 around said axis.

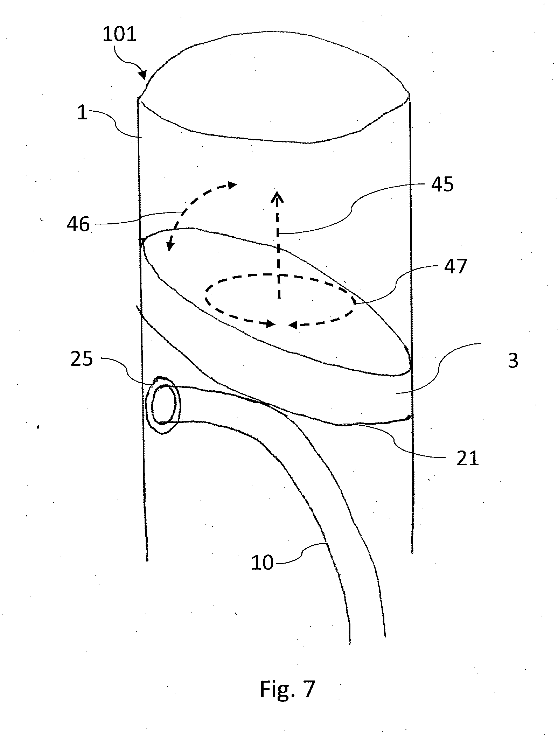

However, the adjustment element 3 can also be pivotable and/or rotatable around a second axis, in particular in circumferential direction of the introducer unit 1 (see Fig. 7).

The adjustment element 3 comprises an electromagnetic actuator 18 as an actuating element. The electromagnetic actuator 18 can be operated from the proximal end region of the apparatus 101 located outside of the patient. The electromagnetic actuator 18 can alter the position of the adjustment element 3 by pivoting the adjustment element around the axis.

An advancement unit 19 schematically depicted in Fig. 4 is formed by a low friction channel 17 on an outer surface of the apparatus 101. The channel 17 allows for manual or mechanical advancement or retraction of the guidewire 10 in its longitudinal direction.

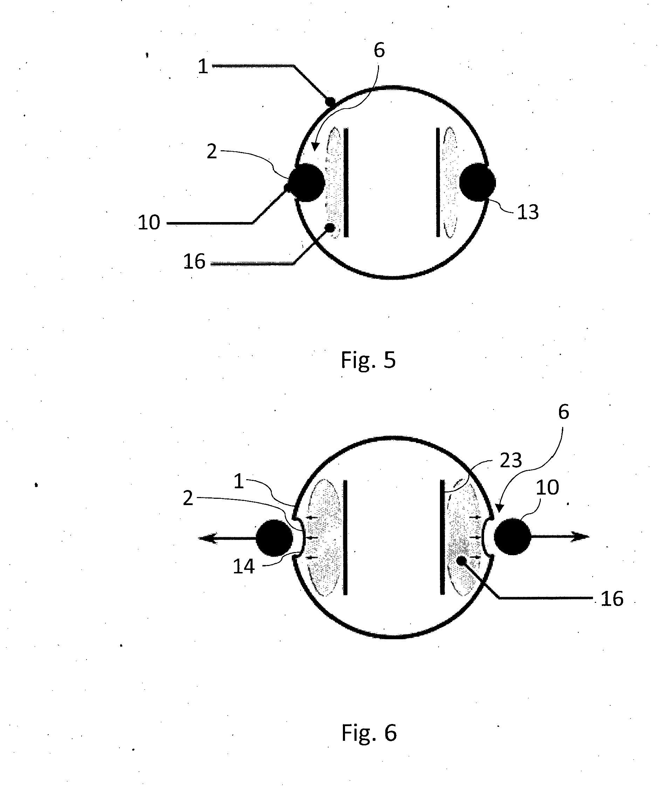

By pushing the guidewire 10 forward, the guidewire 10 can be advanced and guided along the introductory path 38 due to an interaction with the adjustment element 3. Figure 4 shows a deployment member 2 incorporating the channel 17 extending along the longitudinal direction of the introducer unit 1. In this embodiment the low friction channel 17 is only partially enclosed by the lateral wall of the introducer unit 1 in a circumferential direction of the introducer unit 1. The sidewall enclosing the low friction channel 17 functions as a retaining mechanism, which retains the guidewire 10 on the introducer unit 1 in a retaining configuration during introduction into the body duct. During the insertion of the introducer unit 1, the guidewire 10 is held in the low friction channel 17 by being enclosed by the sidewall. As indicated by the dashed arrows in Fig. 4 the guidewire 10 can be deployed in the radial direction by converting the sidewall from the retaining configuration to a release configuration by inflation of a fluid into a deformation area 6 (see also Fig. 5 - 6).

Figure 5 and Fig. 6 show a cross-section plane of an embodiment of an introducer unit 1 with a deformation area 6 in the retaining configuration 13 and release configuration 14, respectively. The deformation area 6 can retain the guidewire on the introducer unit 1 in the retaining configuration by partially enclosing the guidewire 10 in the channel 17 (see Fig. 4 and Fig. 5).

The guidewire 10 can be deployed in the radial direction if the retaining mechanism 6 assumes the release configuration 14 (see Fig. 6). The retaining mechanism is formed in this embodiment by the deformation area 6 which extends over a longitudinal deformation section of the introducer unit 1. The guidewire 10 is deployed from the deployment member 2 by inflation of area an inflatable bladder 16.
bladder 16If the balloon is inflated, the guidewire 10 is ejected outwards through the partial enclosure by the outer surface of the introducer unit 1. The channel 17 in the release configuration is still partially enclosed such that the guidewire 10 cannot re-enter into the channel. In an uninflated state of the bladder, the sidewalls of the channel 17 hinder the guidewire 10 from moving radially outwards (see Fig. 5). A profile of the channel 17 is configured to have essentially the same size as the profile of the guidewire 10. The enclosed guidewire 10 is movable in the longitudinal direction but not in radial direction of the introducer unit 1 (see Fig. 5).

Figure 7 shows a perspective view of an apparatus 101 with an introducer unit 1 comprising an adjustment element 3. The introducer unit 1 also encloses a guidewire 10 which assumes a pre-defined position in relation to an opening 25. The adjustment element 3 has a guiding surface 21 which can be contacted by the guidewire 10 to change its pose and angular deployment orientation. The angular deployment orientation of the guidewire 10 can be altered by the adjustment element 3 by pivoting relative to the longitudinal axis 45 around an axis in a first pivoting movement 46. The adjustment element 3 is also pivotable in a second pivoting movement 47 around the longitudinal axis 45 of the introducer unit 1. Thereby, the guiding surface 21 can assume a desired orientation in such a way that the guidewire 10 can

Figure 8 shows an embodiment of an apparatus 101 with an introducer unit 1 having a wall 34. A guidewire 10 is located within the introducer unit 1. The introducer unit 1 has an opening 25 allowing deploying the guidewire 10 from an inner cavity of the introducer unit 1. The guidewire 10 can be inserted into a branched body duct using the adjustment element (not shown in the figure) by manually advancing the guidewire from a proximal end in the longitudinal direction. Once the guidewire 10 has been longitudinally advanced through the opening 25 into a branching body duct, the apparatus 101 can be retracted. The introducer unit 1 is provided with a retraction recess 20 that is essentially an extension of the opening 25 up to the distal end of introducer unit 1. After deploying the guidewire 10 in the desired branched body duct, the retraction recess 20 can be opened by rotating an enclosing sleeve 23 having a slot 24 such that the slot 24 is circumferentially aligned with the recess 20. Thereafter the apparatus can be retraced without colliding with the guidewire 10.

Figure 9 shows an apparatus 101 with an introducer unit 1 with lateral pivoting panels 33 completely enclosing a deployment member 2 underneath (see dotted lines). The outer surface of the introducer unit 1 hence defines a retaining mechanism 6 which extends over the complete longitudinal length of the introducer unit 1. The retaining mechanism 6 has two pivoting panels 33 which close an opening 25 along the longitudinal section 15 of the deployment member 2. The a panels 33 can convert the retaining mechanism 6 from the retaining configuration to a release configuration. Therefore, the panels 33 are pivotable laterally inwardly such that the outer diameter of the apparatus 101 is not increased by'opening the opening 25 to release a surgical instrument (see Fig. 10). The movement of the panels 33 can be operated from a proximal end of the apparatus e.g.by electromagnetic actuators.

Figures 10 and 11 show a radial cross section of an outer surface of an introducer unit 1 with movable panels 33. Figure 10 shows inwardly movable panels 33 of a wall section of the introducer unit 1, similar as shown in Fig. 9. The two panels 33 are pivotable around a parallel axis to the introducer unit 1. Thereby an opening 25 for deploying and detaching a surgical instrument in a radial direction of the introducer unit 1 can be opened.

The single panel 33 in Fig. 11 is rotatable around an axis parallel to a longitudinal axis of the introducer unit 1 which passes through a centre region of the panel 33. The pivoting movement of the panels 33 in Fig. 10 and Fig. 11 can be controlled and operated by an electromagnetic actuator (not shown in the Figures) to cause pivoting movements of the panels 33 around hinges 40. The openings 25 are sufficiently large to radially deploy surgical instruments.

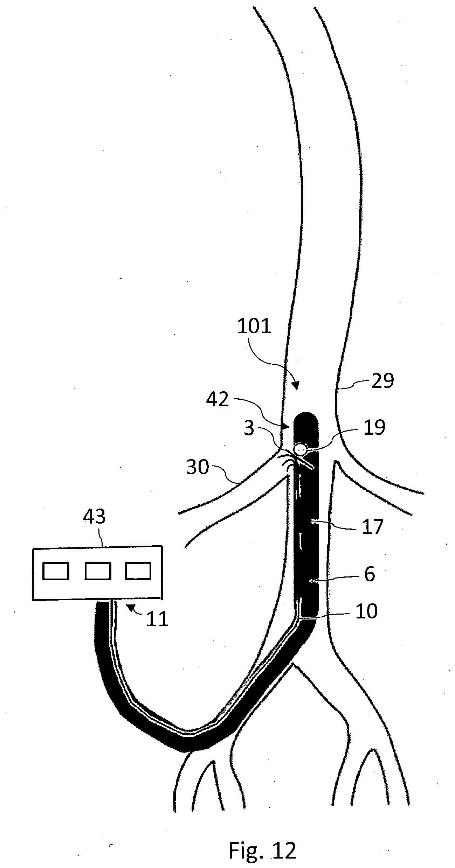

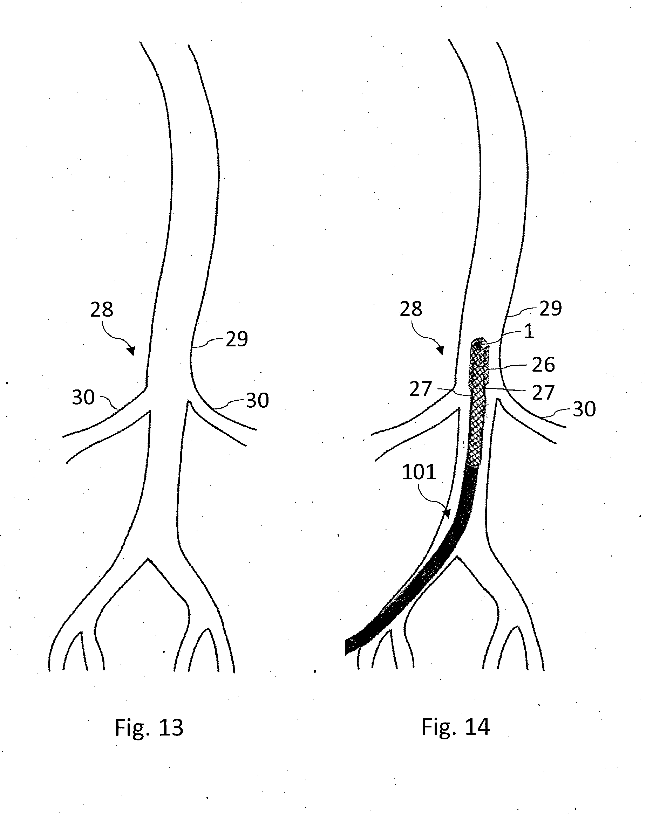

Figure 12 shows the embodiment of the apparatus 101 with an introducer unit 1 according to Fig. 3 and Fig. 4. The introducer unit 1 in Fig. 12 is inserted into the endovascular anatomy of a patient with a main body duct 29 and a branching body duct 30 (see Fig. 13). The introducer unit 1 has a pivotable adjustment element 3 which allows adjusting the deployment orientation of the guidewire 10 into the branched body duct 30 by an electromagnetic actuator 19. The guidewire 10 can be advanced through the low friction elongated channel 17 running from a proximal end 11 to an opening at a radially outer surface of the introducer unit 1 near the distal end 42. At the proximal end 11 of the apparatus 101 there is a computer aided control unit 43 which can be used to operate the electromagnetic actuator 19 and the retaining mechanism 6 comprising the inflatable bladder (see Fig. 5'and 6). In addition, the control unit 43 can be used to mechanically advance and retract the guidewire 10 in longitudinal direction automatically.

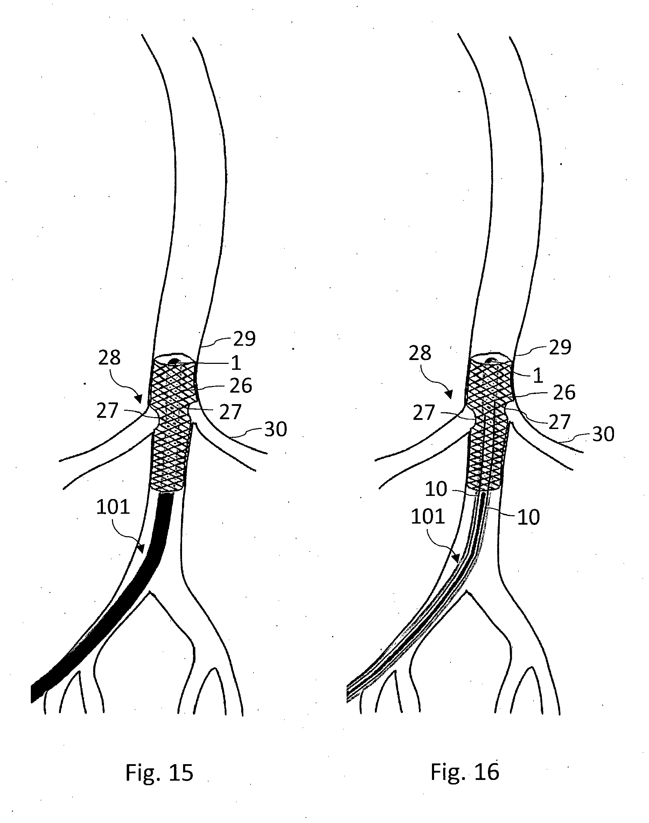

Figures 13 to 18 show the insertion of an apparatus 101 into a branched body duct in order to place a stent 26. Figure 13 shows the endovascular anatomy of the abdominal aorta branched by two renal arteries and two iliac arteries. The introducer unit 1 of the apparatus 101 is introduced loaded with a fenestrated aortic stent 26 as shown in Fig. 14. A stent support (not shown in the figure) can be arranged on the outer surface of the introducer unit 1 and configured to detach and/or deploy the stent 26. Surgical instruments, in particular guidewires 10 are arranged at pre-defined locations, preferably corresponding to the fenestrations 27 of the aortic stent 26 as illustrated in Fig. 15.

The introducer unit 1 is placed completely in the main body duct 29 of the abdominal aorta neighbouring the branching artery body ducts 30. Figure 15 shows the stent 26 in the deployed configuration, preferably frictionally contacting the surrounding main body duct 29. The fenestrations 27 of the stent 26 are arranged at an angular and longitudinal orientation of the introducer unit 1 corresponding to the branching body ducts 30.

Figure 16 shows that the guidewires 10 are pre-positioned according to Fig. 1 and the openings of the introducer unit 1 are pre-positioned accordingly to receive the guidewires 10 at the branching body duct 30 locations. The adjustable elements as shown in the previous figures (not depicted in the figures 14 - 18) allow adjusting the direction of the guidewires 10 such as to meet the branching body ducts. The adjustment element can be adjusted to the angle of the branched body ducts of the renal arteries, in particular to an angle from a longitudinal axis of the introducer unit 1.

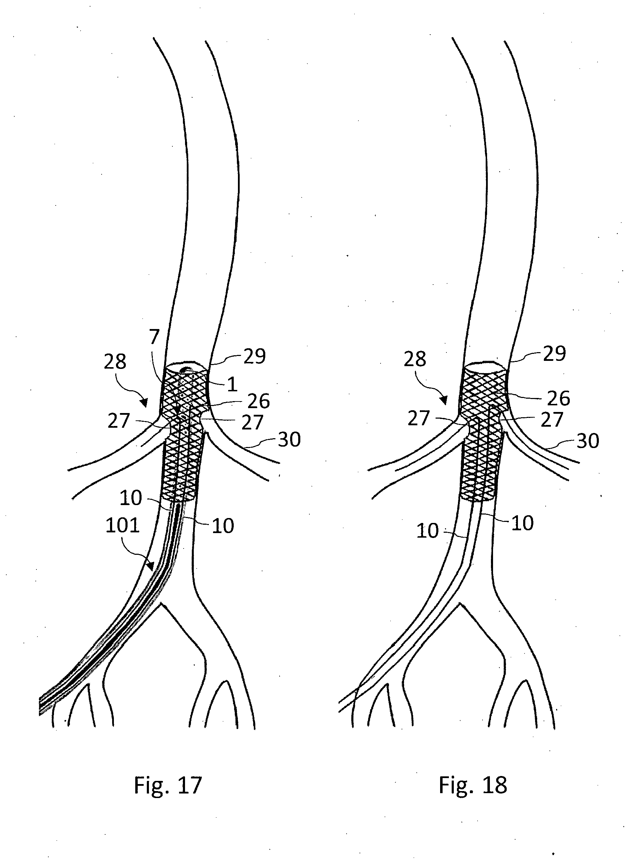

In Fig. 17 and 18 the guidewires 10 are positioned in the branching body ducts 30 by being further moved along their longitudinal direction from the introducer unit 1 through the fenestrations 27 of the stent 26. Here, the guidewires 10 are inserted using one adjustment element sequentially.

Figure 18 shows that the introducer unit 1 was retracted leaving the guidewire 10 in place. A retaining mechanism a describe hereinabove was converted from a retaining configuration to a release configuration and the guidewires 10 were deployed.The guidewires 10 can be used for deploying additional stent components of the stent 26.

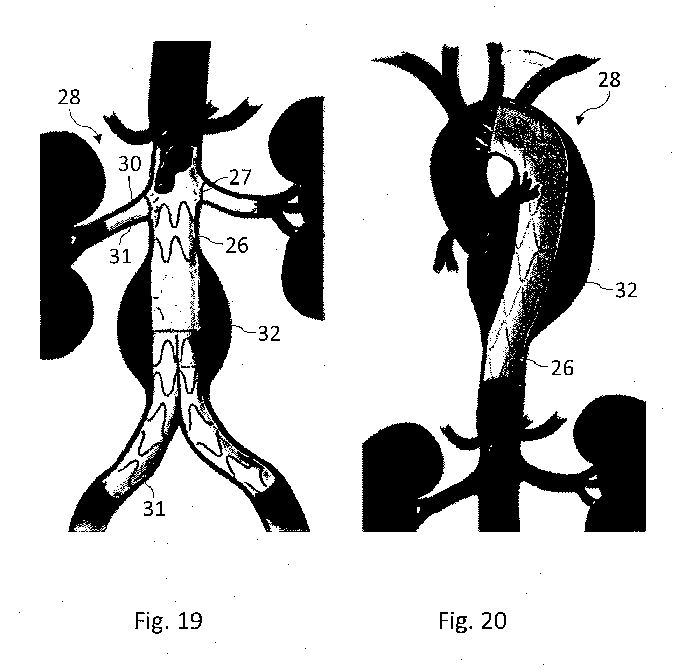

Figure 19 and Fig. 20 show stents 26 which could be placed in branched body ducts using embodiments of an introducer unit according to the invention. In particular, further stent components 31 to be connected to the stent 26 can be introduced into branching body ducts 30 with the guidewires 10 w. The stent 26 can be configured to extend over aneurysms 32.

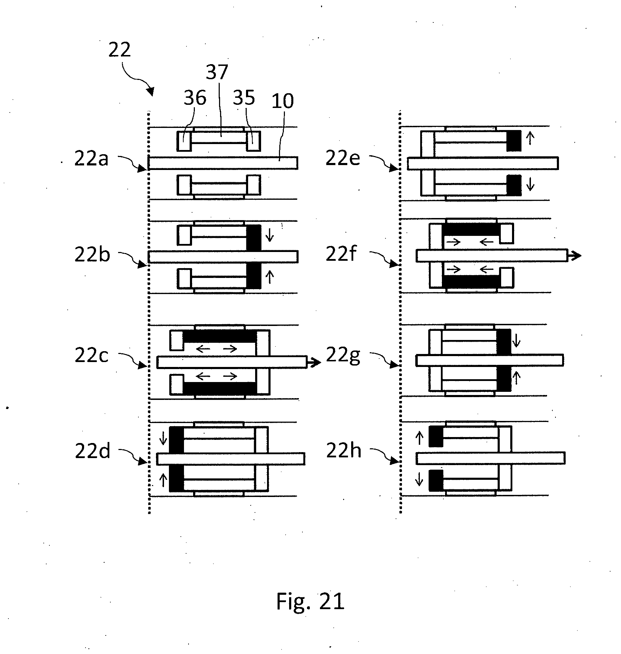

Figure 21 shows a schematic illustration an inchworm device 22 for advancing and retracting the guidewire along its longitudinal direction. The device 22 is arranged within an apparatus 101 as previously described.

The inchworm mechanism is powered by a piezoelectric mechanism. In a relaxed state 22a the inchworm mechanism does preferably not contact the guidewire 10. The guidewire 10 can be advanced forward by frictionally contacting the guidewire 10 in step 22b with a first contacting element 35 of the inchworm mechanism and expanding an expandable element 37 of the inchworm mechanism in longitudinal direction of the guidewire 10 in step 22c. Thereafter, the guidewire 10 can be frictionally engaged by a second contacting element 36 of the inchworm mechanism in addition to the first contacting element 35 in step 22d. The first contacting element 35 then disengages the guidewire 10 and the expandable element 37 is contracted in longitudinal direction in step 22f. In step 22g the first contacting element frictionally contacts the guidewire 10 again and in the final step 22h the second contacting element 36 disengages the guidewire 10.

## Claims

1. Apparatus (101) for delivery of at least one surgical instrument (10), in particular at least one guidewire, into bifurcated body ducts (28) of a patient, the apparatus comprising an introducer unit (1) with a deployment member (2) for deploying the surgical instrument (10),
the deployment member (2) having at least one deflection section (7) for deploying, in particular radially deploying, said at least one surgical instrument (10) along an introductory path (38) into a branching body duct (30.),
**characterized in that** the apparatus (101) comprises an adjustment element (3) configured to adjust an angular deployment direction (8) of said surgical instrument (10) at the deflection section (7) and to alter a pose of said at least one surgical instrument (10) with respect to the introducer unit (1).

2. Apparatus according to claim 1, wherein the adjustment element (3) has a guiding surface (21) which is arrangeable in at least two different poses for guiding said at least one surgical instrument (10).

3. Apparatus according to one of the preceding claims, wherein the guiding surface (21) of the adjustment element (3) is configured to be pivoted around a first axis such as to change its angle relative to the longitudinal axis of the introducer unit (1) at the deflection section (7), the adjustment element (3) optionally being configured to be pivoted along a second axis in a different plane than the first axis of the introducer unit (1).

4. Apparatus according to one of the preceding claims, wherein the adjustment element (3) is provided with an actuating element (18) selected from the group of (i) a microfluidic unit, (ii) an electromagnetic actuator, (iii) a micro-electro-mechanical system, (iv) a retractable pull-wire, (v) a volume adjustment unit, and (vi) a piezoelectric element.

5. Apparatus according to one of the preceding claims, wherein the deployment member (2) comprises an advancement unit (19) for advancing the surgical instrument along an axis of the introducer unit (1),
the advancement unit (19) preferably being selected from the group of
(i) an inchworm device (22) or
(ii) a low friction elongated channel (17) leading from a proximal end region (11) of the apparatus (101) to at least the deflection section (7) of the apparatus (101), the apparatus preferably further having a manual or mechanical insertion unit which is configured for insertion of surgical instruments along said elongated channel (17).

6. Apparatus according to one of the preceding claims, wherein the apparatus (101) comprises a retaining mechanism (6) configured to releasably retain at least a section of said surgical instrument (10) at the introducer unit (1).

7. Apparatus according to claim 6, wherein the retaining mechanism (6) is convertible from a retaining configuration (13) to a release configuration (14), wherein
in the retaining configuration (13) the retaining mechanism (6) is configured such that an elongated longitudinal section (15) of the deployment member (2) is at least partially closed in a circumferential direction of the introducer unit (1), and
in the release configuration (14) the retaining mechanism (6) is configured such that the longitudinal section (15) of the deployment member (2) is at least partially open such that at least a portion of the surgical instrument (10) confined within a cavity of the deployment member (2) is detachable from the introducer unit (1) in a radial direction of the introducer unit (1) through the longitudinal section (15) of the deployment member (2) where it is partially open.

8. Apparatus according to claim 6 or 7, wherein the retaining mechanism (6) is convertible from the retaining configuration (13) to the release configuration (14) by at least one of
(i) moving, preferably rotating around the longitudinal axis, at least one wall member (34) of the introducer unit (1) which closes an opening in the longitudinal section (15) of the deployment member (2) or the introducer unit (1),
(ii) pivoting a panel (33) closing an opening in the longitudinal section (15) of the deployment member (2) of the retaining mechanism (6) about an axis,
(iii) translationally moving at least one panel (33) closing an opening in the longitudinal section (15) of the deployment member (2) relative to the introducer unit (1).

9. Apparatus according to one of the claims 6 to 7, wherein the retaining mechanism (6) has a deformation area (16) for retaining the instrument,
wherein the deformation area (16) is deformable, in particular elastically deformable and wherein
the retaining mechanism (6) is convertible from the retaining configuration (13) to the release configuration (14) by applying pressure to the deformation area (16).

10. Apparatus, according to one of the preceding claims, wherein the deflection section (7) has an opening (25) in a lateral wall of the introducer unit (1) allowing the deployment of said at least one surgical instrument (10) from an inner cavity of the deployment member (2) along the introductory path (38),
wherein a section of the introducer unit (1) arranged distally from the opening (25) preferably provides a retraction recess (20) running in a longitudinal direction and having a greater circumferential profile than the circumferential extensions of the surgical instrument (10).

11. Apparatus according to one of the preceding claims, wherein the apparatus (101) comprises at least two deployment sections (7) which are positioned at different circumferential positions of the introducer unit (1) and/or at different longitudinal positions of the introducer unit (1).

12. Apparatus according to one of the preceding claims, wherein the apparatus (101) comprises at least one radiopaque marker, preferably arranged at the deployment section (7).

13. Apparatus according to one of the preceding claims 6 to 12, wherein the surgical instrument (10) is pre-mounted in the retaining mechanism (6) at a predetermined position relative to the deployment section (7) or a plurality of surgical instruments (10) are pre-mounted at a predetermined position relative to a respective deployment section (7) associated to each surgical instrument.

14. Apparatus according to one of the preceding claims, wherein the apparatus (101) comprises a stent (26) mounted on a stent support, the stent having fenestrations (27) aligned with the deployment section (7).

15. Apparatus according to claim 14, wherein the surgical instrument (10) is arranged and/or the at least one opening (25) is arranged in a pre-defined position in relation to the fenestrations (27) of the stent (26).

16. A method for delivering at least one surgical instrument (10) into bifurcated body ducts (28) of a patient, optionally using an apparatus (101) according to any one of the preceding claims, comprising the following steps:
- Optionally pre-defining a location of said surgical instrument (10), preferably a plurality of surgical instruments (10), and/or at least one opening (25) in a lateral wall of an introducer unit (1) with respect to fenestrations (27) of a stent (26) placed or to be placed adjacent the bifurcated body duct (28);
- Inserting the apparatus (101) into a patient to a site adjacent a bifurcation of the body duct (28);
- Adjusting an angular deployment orientation of said surgical instrument (10) at a deployment section (7);
- Deploying, preferably radially deploying, said surgical instrument (10) using a deployment member (2) along an introductory path.(38) along a body duct (30) branching from the body duct in which the introducer unit (1) is positioned;
- Optionally detaching said surgical instrument (10) by releasing a retaining mechanism (6);
- Retracting the apparatus (101) from the body duct relative to the surgical instrument which remains at least partially within the branched off body duct (30).
